# EUROPEAN PATENT SPECIFICATION

(11) **EP 1 048 952 B1**
(45) Date of publication and mention of the grant of the patent: **26.03.2003**
(21) Application number: 99201361.5
(22) Date of filing: 29.04.1999
(51) Int. Cl.: G01N 33/58, G01N 33/50, G01N 21/64

(54) **Determining analyte mobility**
Bestimmung von Analytbeweglichkeit
Détermination de la mobilité d'un analyte

(43) Date of publication of application: 02.11.2000
(73) Proprietor: Erasmus Universiteit Rotterdam, 3015 GE Rotterdam (NL)
(72) Inventor: Houtsmuller, Adriaan Barend, 1072 XA Amsterdam (NL); Vermeulen, Willem, 3333 VL Zwijndrecht (NL)
(74) Representative: Ottevangers, Sietse Ulbe

(56) References cited:
- WO-A-91/07651
- WO-A-92/00796
- WO-A-97/11355
- WO-A-98/45704

## Description

The invention relates to the field of measuring or determining mobilities of analytes or molecule(complexes).

Measurement of mobilities of analytes in liquid systems or at interfaces (such as membranes) between liquid systems or liquid and solid systems is of great importance for understanding a number of dynamic phenomena relating to chemical and physical reactions, be it of organic or inorganic nature.

Examples of these are chemical reactions wherein one or more molecules or analytes undergo chemical change or react with each other, diffusion or transport of analyte in a liquid as a function of the viscosity and size of the analyte, mobility of analyte adsorbed to an interface, transport over an interface, and desorption from and readsorption onto a solid surface. Yet other examples comprise determining the fate and direction of molecular cascade reactions in complex liquid systems, by which one chemical reaction may or may not lead to a cascade of subsequent chemical reactions. In particular, such complex chemical or cascade reactions can be found in (living) cells.

A well-known method for measuring mobilities driven by diffusion is for example fluorescence photobleaching recovery, also known as fluorescence recovery after photobleaching (FRAP) (Axelrod et al., Biophysical Journal, 16:1055-1069, 1976; Blonk et al., J. Micr. 169:363-374, 1993; Reits et al., EMBO 16:6087-6094, 1997). A prerequisite of the FRAP method is that the mobile analyte is fluorescent or carries a fluorescent label. Herein, in a defined or set volume of the sample, the fluorophores are in general irreversibly bleached, (loose fluorescent properties) by a short and intense light pulse. After bleaching, the non-fluorescent analyte in the observed region may be replaced by diffusion of other fluorophores, resulting in recovery of the fluorescent signal. The rate of recovery of the fluorescent signal is a measure for the mobility of the analyte in the system. The amount of recovery is a measure of the fraction of the analyte that is mobile in the observed region. FRAP can be for example performed within a conventional microscope. A laser beam is focused into a specimen placed on the stage of a light microscope. The laser may be used for excitation of the fluorochrome as well as for bleaching. A device may be used to switch between low laser intensity for measuring and high laser intensity for bleaching. A photomultiplier may be used for measuring the intensity of the fluorescent signal.

WO-A-92/00796 discloses a method and apparatus for determining the electrophoretic mobility of fluorescent-labeled molecules (DNA) by measuring the fluorescence distribution after photobleaching, wherein said photobleaching is achieved by exposing a spot of the sample contained in a capillary tube to a 3W argon-ion laser beam. WO-A-98/45704 discloses a drug screening method based on the observation of variations of intracellular signal pathways (molecule mobility) by measuring changes in fluorescence distribution in cells after exposing said cells to a light source (a 100W HBO arc lamp).

In general, current methods determining analyte mobility, albeit allowing studying the phenomenon of mobility and diffusion rates in general, do not or only little allow studying the effects that external or internal stimuli or inhibitors have on said chemical reactions or diffusion processes. In particular, studying dose-response relationships between the addition of chemical or physical agents (either stimulating or inhibiting said reactions or processes, such as catalysts for organic or inorganic reactions), or studying effects of such agents in themselves on analyte mobility is hardly possible, often the required sensitivity, specificity or precision of the method used is inadequate. Also, it is often not possible to discern the behaviour of a (transiently) immobile analyte fraction from the behaviour of a (very) slow analyte fraction, again for example not allowing dose-response studies.

The invention provides a method for determining the mobility of a certain analyte in a set volume comprising labelling said analyte with a laser-inducible moiety and subjecting a spot or area in a pre-determined plane through said set volume to a focused laser beam of relatively low intensity for a relatively long time thereby inducing a measurable change in said moiety when exposed to said laser beam and determining the distribution of laser-exposed moiety in said set volume, preferably in said plane, preferably using a microscope. Such a microscope is preferably equipped with items such as a motorised scan stage, software and hardware or a computer to apply quantitative detection techniques, such as quantitative fluorescence detection techniques, or analyse the data, digital cameras to provide a digital image when so required, and additional computers to store and retrieve data, optionally in real time.

Contrary to for example standard or conventional FRAP technology, where use of a pulse of short duration and high intensity is in general applied, the invention provides the insight that for accurate, more specific, more sensitive or more precise determination of analyte mobility a pulse of relative long duration and relatively lower intensity is preferred. Furthermore, conventional FRAP in general, after the fluorophores in said region have been bleached by said short and intense light pulse, detects returning fluorophores which replace the bleached fluorophores by diffusion to said laser-exposed region, resulting in recovery of the fluorescent signal. The rate of recovery of the fluorescent signal from said region is in general a measure of the mobility of the analyte in the system.

However, contrary to for example conventional FRAP, in a method as provided by the invention, bleach pulses of lower intensity, which in the case of excitation at 488nm with a 60 mW Ar-laser use a neutral filter passing <50%, preferably <20%, more preferably <10% or even <5% of the laserlight; (when using another lasertype and/or other filter, one adjusts these settings accordingly) and longer duration such that a period of time is used that, at the filter setting used, is required to bleach ^{∼}30-70 of the mobile labelled molecules in said set volume) are used. Herewith, a larger proportion of the mobile analyte fraction is exposed to the laser beam and the contribution of the immobile fraction to the measured signal is increased, allowing more accurate determination of the ratio of immobile to mobile fraction.

In a preferred embodiment the invention provides a method wherein said moiety is a fluorescent moiety; such moiety may be detected after laser exposure has excited said fluorescent moiety, thereby allowing detection of the laser-exposed moiety by the signal it emits, however, more preferred is a method as provided by the invention wherein said fluorescent moiety, after earlier excitation, bleaches upon exposure to said laser beam. Examples of fluorescent moieties are well known in the art. Analytes, such as proteins, nucleic acid molecules, enzyme-cofactors, organic carbohydrate polymers such as cellulose, amylose or amyolopectine, carbohydrates per se, fats or fatty acids, can for example be labelled with fluoresceine isothiocyanate or any other fluorophore well known in the art. In particular the invention provides labelling of proteins through genetic modification of nucleic acid encoding said protein whereby said protein is fused, through processes of transcription and translation, or labelled with a fluorophore. One well known example of such a fluorophore is green fluorescent protein (GFP), others are yellow fluorescent protein (YFP), blue fluorescent protein (BFP), cyanic fluorescent protein (CFP) or other variants of fluorescent protein that allow fusing or labelling proteins during transcription and translation from recombinant nucleic acid.

In a preferred embodiment, the invention-provides a method for determining the mobility of a certain analyte in a set volume comprising labelling said analyte with a laser-inducible moiety and subjecting a spot in a pre-determined plane through said set volume to a focused laser beam of relatively low intensity for a relatively long time thereby inducing a measurable change in said moiety when exposed to said laser beam and determining the distribution of laser-exposed moiety in said plane wherein said distribution is determined relative to the distance of said laser-exposed moiety to said spot. Taking the distance into account allows better calculation of diffusion rates of detected analyte, whereby diffusion rates of course also depend on the molecular weight or size of the analyte in question. Changes in molecular weight, and thus in analyte mobility, induced for example by the chemical reactions studied, can thus also be determined by a method as provided by the invention.

Distribution is for example determined in one or more concentric rings in said plane around said spot or area where the laser beam has been focused. Of course, calculations as to distributions are best done on the basis of digital images provided by computerised analyses of images from said microscope.

In a preferred embodiment, the invention provides a method wherein said distribution is determined a relatively long time after laser-exposure of said moiety, to allow for diffusion of mobile analyte through said set volume.

The invention furthermore provides a method wherein said spot is subjected to a substantially stationary confocal laser beam. Holding the beam stationary, or allowing only little movement, compared with for example scanning a region of said plane even more increases the sensitivity, specificity and precision.

Furthermore, the invention provides a method wherein said set volume is exposed to said laser beam prior to subjecting said spot in said plane through said set volume to a focused laser beam. For example, when FRAP technology is used, a prebleach measurement or image is thus obtained that can be compared with other measurements to for example provide a control or null measurement, to facilitate calculation. Also, the invention provides a method according to the invention wherein said set volume is exposed to said laser beam after subjecting said spot in said plane through said set volume to a focused laser beam. For example, when FRAP technology is used, a postbleach measurement or image is thus obtained that can be compared with other measurements, to facilitate calculation.

In a further preferred embodiment, the invention provides a method wherein the analyte studied comprises an organic molecule or the chemical reactions comprises organic chemistry. Organic molecules, as is known in the art vary widely. Central to life, where organic reactions lay at the heart of the matter, are for example fatty acids, e.g. constituting membranes around cells and cell organelles, carbohydrate and polymers thereof being building blocks and energy storing components of organisms, nucleic acids, carrying genetic information for proteins and proteins themselves, which are often central to performing or regulating chemical reactions in a cell or cell organelle. For example, in a detailed description of the invention as provided below, the study of protein mobility is further explained.

A method according to the invention is preferably applied in a set volume, such as a droplet, mounted or placed in a suitable position for microscopic evaluation, however, application of the invention to relatively large volumes is also provided. In a preferred embodiment, said set volume is contained in a container, such as a (miniaturised) reaction vessel, microtitre well, or other suitable container. In a preferred embodiment, said set volume is contained in a cell or cell organelle. Said cell or cell organelle can be either living (essential functions are still performed) or dead (fixation with fixatives known in the art is in general sufficient to kill such a cell or cell organelle). All cells are in essence suitable to be studied using a method as provided by the invention, be it an individual cell that is studied as single entity, or be it a cell in a tissue, organ, or organism. Also, the invention provides a method according to the invention wherein analyte mobility is determined in at least two cells, for example contained in said tissue, organ or organism. Suitable tissues, organs or organisms are those that in general are studied by for example pathologists, histologists or biologists, whereby it is preferred to limit the scale or size of the tissue, organ or organism somewhat to for example the size of a mammalian embryo, a nematode such as *C. elegans*, or other suitable organisms known in the art. In a preferred embodiment of the invention, such cells (individually or contained in tissue, organ or organism) are transiently or stably transfected, transformed or otherwise genetically modified with a recombinant nucleic acid encoding a protein or analyte of interest.

In a preferred embodiment, the invention provides a method for determining the mobility of a certain analyte in a set volume comprising labelling said analyte with a laser-inducible moiety and subjecting a spot in a pre-determined plane through said set volume to a focused laser beam of relatively low intensity for a relatively long time thereby inducing a measurable change in said moiety when exposed to said laser beam and determining the distribution of laser-exposed moiety in said plane said method further comprising determining the mobility of said certain analyte in at least one other set volume or container, thereby allowing comparing mobilities under different circumstances set to each volume or container. Preferable, in at least one container said analyte is rendered at least partly immobile, suitable immobilisation is for example obtained by freezing, radiation, fixation with fixatives, and other methods known in the art. In cells or cell organelles, such analytes are preferably rendered immobile by fixation, using fixatives known in the art.

Furthermore, the invention provides a method wherein-the distribution of laser-exposed moiety is determined relative to the pre-exposure distribution of said moiety. Studying pre-exposure distribution adds even more to the understanding of mobility processes and diffusion events in said set volume, and facilitates studying mobility in time. In addition, the invention provides a method according to the invention wherein the distribution of laser-exposed moiety is determined relative to post-exposure time, thereby facilitating discerning the behaviour of a (transiently) immobile analyte fraction from the behaviour of a (very) slow analyte fraction.

The invention also provides a method according to the invention determining the mobility of at least one other analyte comprising labelling said other analyte with a second moiety. Such second moiety is preferably of another nature that said first moiety, when said first is for example CFP, said second can be for example another fluorescent protein.

Furthermore, the invention provides a method to determine analyte mobility in real time, whereby data are obtained, analysed and provided through rapid computer analyses, thus facilitating studying analyte mobility when circumstances are varied during analysis time.

The invention provides a method for determining analyte mobility, by studying the phenomenon of mobility and diffusion rates as provided by the invention that allows studying the effects that external or internal stimuli or inhibitors have on said chemical reactions or diffusion processes. In particular, studying dose-response relationships between the addition of chemical or physical agents (either stimulating or inhibiting said reactions or processes, such as catalysts for organic or inorganic reactions), or studying effects of such agents in themselves on analyte mobility is provided, the sensitivity, specificity or precision of the method as provided by the invention is sufficiently large to do so. It is now also possible to discern the behaviour of a (transiently) immobile analyte fraction from the behaviour of a (very) slow analyte fraction, again allowing dose-response studies. For example, in the detailed description, the mobility of a DNA repair protein analyte upon UV-radiation leading to DNA damage in a cell is provided.

In one embodiment, the invention for example provides a method for testing biological activity of a candidate drug comprising subjecting a cell to said candidate drug and testing analyte mobility in said cell. Said method preferably comprises testingisaid mobility with FRAP-(fluorescence redistribution after photobleaching) technology, preferably with a method as provided by the invention, allowing testing dose-response relationships. Examples of such a method as provided by the invention are for example to screen candidate antagonists of hormone (such as steroid hormone) receptor functioning applying FRAP technology, preferably as provided by the invention using various agonists and antagonists and a combination of agonist and antagonist as stimulus to cells (transiently or stably) transfected with cDNA of fluorescent protein fused hormone receptors or cells microinjected with fluorescently labeled hormone receptors; likewise to screen candidate activators of any signal transduction pathway that results in binding of any proteins to a certain immobile or slowly diffusing cellular component, with cells (transiently or stably) transfected with cDNA of fluorescent protein fused 'signal induced binding protein' or cells microinjected with fluorescently labeled 'signal induced binding protein'; to screen candidate substances that interfere with a signal transduction pathway that results in binding of a protein to some immobile or slowly diffusing cellular component, with cells (transiently or stably) transfected with cDNA of fluorescent protein fused 'signal induced binding protein' or cells microinjected with fluorescently labeled 'signal induced binding protein'; to screen possible DNA-damaging agents with a range of cells (transiently or stably) transfected with cDNA of fluorescent protein fused variations of different DNA-repair proteins involved in the repair of different types of damage or ranges of cells microinjected with different fluorescently labeled DNA-repair protein involved in different repair of different types of DNA-damage. In addition, it is now possible as provided by the invention to determine the percentage of a DNA-repair protein that becomes immobilised as a result of induction of DNA-damage by any DNA-damaging agent in varying concentrations or intensities by applying a method according to the invention to cells (transiently or stably) transfected with cDNA of fluorescent protein tagged DNA-repair protein or cells microinjected with fluorescently labeled DNA-repair protein or to determine the duration of immobilisation of a proteinthat becomes (transiently) immobilised as a result of a stimulus (e.g. induction of DNA-damage by a DNA-damaging agent) by applying a method according to the invention to cells (transiently or stably) transfected with cDNA of fluorescent protein tagged DNA-repair protein or cells microinjected with fluorescently labeled DNA-repair protein.

In yet another embodiment, the invention provides a method for testing activity of a candidate catalyst, for example for inorganic or organic chemical reactions, comprising subjecting a set volume of a liquid system, i.e contained in a reaction vessel or other suitable container wherein said chemical reaction is studied, to said candidate catalyst and testing analyte mobility in said set volume. It is provided by the invention to test said mobility with FRAP-(fluorescence redistribution after photobleaching) technology, preferably using a method according to the invention. Examples of such a method as provided by the invention comprise catalyst reactions wherein said catalyst is an enzyme. It is for example provided to test polymerisation or degradation of polymers under influence of a catalyst, e.g. an enzymatic catalyst. Since diffusion rates, and thus differences of mobility, are related to molecular weight, polymer forming or degradation can be studied by a method as provided by the invention by studying mobility change upon candidate catalyst addition. The invention is further explained in the detailed description without limiting the invention.

### Detailed description.

A further stepwise description of an automated method for calculation of the mobile and immobile fraction of fluorescent molecules in a set volume (as exemplified here by a cell), wherein the steps are performed by computer.

### A

1) Make a confocal digital image of the object preferably using relatively low laser power (eg. in the case of excitation at 488nm with a 60 mW Ar-laser use a neutral filter passing <50%, preferably <20%, more preferably <10% or even <5% of the laserlight; when using another lasertype and/or other filter, one adjusts these settings accordingly)(step 1) results in an image that is also called prebleach image).
2) Position the laser focus in the centre of the object and illuminate with stationary laser focus at low laser power for a relatively long period of time (optimally, a period of time is used that is required to bleach ^{∼}30-70 or 40-60% of the mobile labelled molecules). In general this may take 1 to 30 seconds, or longer or shorter depending on laser-power and diffusion rate of the molecules. The combination of long illumination period and low laser power (eg. in the case of excitation at 488nm with a 60 mW Ar-laser use a neutral filter passing <50%, preferably <20%, more preferably <10% or even <5% of the laserlight; when using another lasertype and/or other filter, one adjusts these settings accordingly) facilitates improved measurement of immobile fraction, since a larger proportion of mobile molecules is bleached and therefore the contribution of the signal coming from the immobile fraction is increased.
3) Wait for a definable period of time. For immobilisation measurements a period approximately as long as the illumination time in 2) is preferred. For measurement of average binding time (see below: detailed description B) increasing periods of time are used.
4) Make a confocal digital image of the bleached object (postbleach image), preferably under the same or comparable conditions as the prebleach image was taken.
5) Align the pre- and postbleach images when needed to correct for cell movement and instrumental instability.
6) Make a digital image in which each pixel I(x,y) = [Post (x,y)-background]/[Pre(x,y)-background].
7) Divide the resulting image in concentric rings (annuli) with standard thickness (4 pixels) and increasing diameter. The centre is the position where the laser was focused during bleaching.
8) Calculate the mean fluorescence ratio in each of the rings.
9) Make a plot or table of the fluorescence intensity ratio as a function of distance to the laser spot.
10) Apply step 1-8 to at least one cell and make a plot or table of the mean fluorescence intensity ratio of cells when more than one is tested.
11) Apply step 1-9 to (a) fixed cell(s): all fluorescent molecules are immobile and the resulting curve is a reference curve for 100% immobile molecules.
12) Apply 1-9 to living untreated cells: this provides a reference curve for an untreated situation: often all molecules are mobile and then the curve serves as an all mobile reference curve).
13) Apply 1-9 to living cells to which some stimulus has been given (addition of candidate drug or catalyst to the medium, UV-irradiation to damage DNA, etc.).
14) Calculate the immobile fraction p in living treated cells by fitting (eg. ordinary least squares) the reference curve (from step 12) to the weighted sum of the reference curves from 100% immobile (step 10) and 0%immobile (step 11): *p* * curve(100%imm) + (1-*p*) * curve(0%imm). If living untreated cells cannot be used for a 0% immobile reference curve, the horizontal line through the (estimated) intersection of the 100% immobile reference curve and the curve of the untreated cells is used.

### B. Measurement of average duration of binding:

Perform step 1-9 on living treated cells at increasing the redistribution time in step A3). Intersection of the resulting plot of measured immobile fraction to time is a good estimate of the average binding time. If the resulting plot in not linear, the apparent immobile fraction is in fact a very slowly diffusing fraction.

### Application of a method as provided by the invention to determine protein mobility.

To study the nuclear organization and dynamics of nucleotide excision repair (NER), the endonuclease ERCC1/XPF was tagged with green fluorescent protein and its mobility monitored in living Chinese hamster ovary (CHO) cells. In the absence of DNA damage, the complex moved freely through the nucleus, with a diffusion coefficient (15 ± 5 µm²/s) consistent with its molecular size. Ultraviolet light-induced DNA damage caused a transient dose-dependent immobilization of ERCC1/XPF, likely due to engagement of the complex in a single repair event. After four minutes, the complex regained mobility. These results suggest that (i) NER operates by assembly of individual NER factors at sites of DNA damage rather than by pre-assembly of holocomplexes and (ii) that ERCC1/XPF participates in repair of DNA damage in a distributive fashion rather than by processive scanning of large genome segments.

Nucleotide excision repair (NER) is a sophisticated DNA repair mechanism, that eliminates a wide range of structurally unrelated DNA-lesions (*1*). The most common types of DNA damage targeted by NER are cyclobutane pyrimidine dimers (CPDs) and (6-4) photoproducts (6-4PPs) both produced by the ultraviolet (UV) component of sunlight (1). The major NER pathway operates across the genome (global genome NER), whereas transcription-coupled NER preferentially repairs lesions that block transcription (*2*). Genetic defects in NER are associated with three photosensitive diseases: xeroderma pigmentosum (XP), Cockayne syndrome, and trichothiodystrophy (*3*). Global genome NER is initiated by the DNA damage binding factor XPC/hHR23B (*4*), and transcription-coupled NER by RNA polymerase stalled at a lesion (2). TFIIH, XPA and RPA (5,6) are then recruited resulting in local opening of the DNA helix, proper orientation of the NER machinery, and stabilization of the opened DNA intermediate. The single strand binding protein (RPA), bound to the non-damaged DNA strand helps to position (6) the structure-specific endonucleases XPG (which makes the 3' incision) (7) and the ERCC1/XPF complex (which makes the 5' incision) (*8*). After excision of the damage, the resulting gap is filled in by DNA polymerase δ/ε and auxiliary factors (*1,9*).

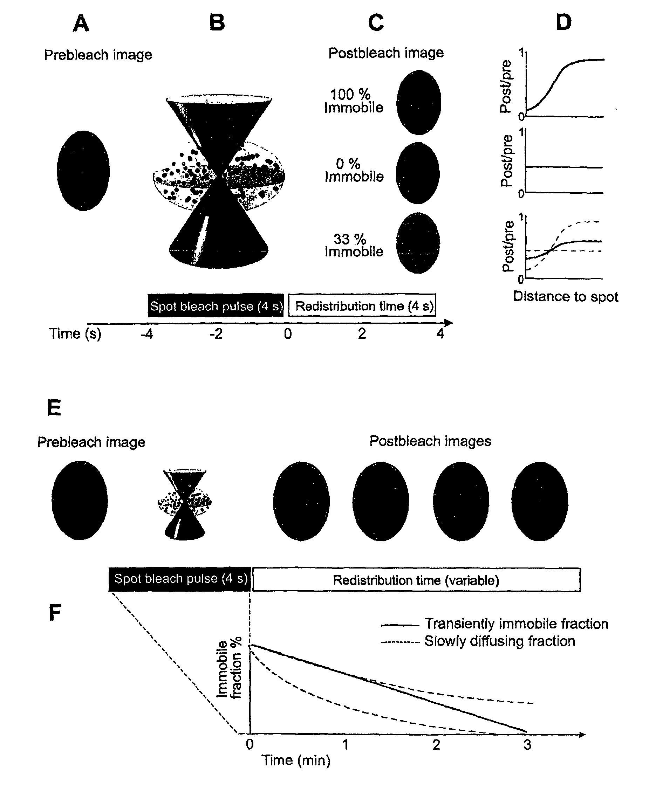

It is unknown whether in intact cells NER operates as a preassembled holocomplex *(10)* or by consecutive assembly of subcomplexes (*11*) or individual NER factors at sites of DNA damage. It is also unclear whether NER proteins find DNA damage by processive scanning of large genome segments or in a distributive fashion by diffusion. To address these questions, we fused the green fluorescent protein (GFP) (12) to the ERCC1 subunit of the 5'-endonuclease ERCC1/XPF *(13)* so that we could study repair in living cells. The fusion gene (ERCC1-GFP) was stably transfected into ERCC1-deficient Chinese hamster ovary (CHO) cells (43-3B) (Fig. 1D). Immunoblot and immunoprecipitation analyses showed that ERCC1 was detectable only in the GFP-tagged form (Fig. 1A) and was expressed at the same level as endogenous ERCC1 in repair-competent HeLa cells and 43-3B cells corrected by natural *ERCC1* (Fig. 1A). The ERCC1-GFP associated with XPF (Fig. 1B); formation of this complex has been shown to be required for the stabilization of both proteins (*8, 14*). Transfection of ERCC1-GFP fully corrected the extreme UV-sensitivity (and other repair defects)(15) of 43-3B cells (Fig. 1C). These data demonstrate that the fusion protein was expressed at physiological levels, was complexed with XPF and its function was not affected by the GFP tag.

Confocal microscopy of living 43-3B cells revealed a predominantly homogeneous nuclear distribution of ERCC1-GFP/XPF, although 26 ± 7% of the nuclei contained 1 to 3 bright fluorescent spots (Fig. 1E). After induction of DNA repair by UV exposure [8 Joules per square'meter (J/m²)], we did not detect any significant alterations in the homogeneous fluorescence pattern, whereas the number of nuclei containing spots decreased to 9 ± 2%. These observations contrast with previous work using immunofluorescence or repair patch labeling showing nonrandom distribution of NER proteins or sites of repair in the nucleus (*16,17*). The function of the bright fluorescent spots is unclear. They were not artifacts of GFP-tagging or ERCC1-GFP overexpression as they were also detected by anti-ERCC1 antibodies in 30 to 40% of HeLa cells expressing endogenous ERCC1 (Fig. 1F). Since the spots are present in a fraction of the cells, a general role in enzyme storage is not likely.

To determine whether ERCC1 is mobile or fixed, we studied the nuclear mobility of ERCC1-GFP/XPF using fluorescence redistribution after photobleaching (FRAP) technology (*18,19*). The diffusion coefficient (*D*) was determined by measuring the movement of fluorescent ERCC1-GFP/XPF into a previously bleached strip through the nucleus, as described before (*19*). The method was first calibrated with cells expressing enhanced-GFP (EFGP) *(13),* and the measured D value (58 ± 9 µm²/s) was in the range of a previously reported value (19).

In the absence of DNA damage, ERCC1-GFP/XPF moved freely through the nucleus of CHO cells. The measured diffusion coefficient (15 ± 5 µm²/s) was consistent with the size of ERCC1-GFP/XPF (163 kD)(*20*), suggesting that the majority of ERCC1/XPF is not part of a large NER holocomplex. Free mobility was also found for other NER proteins tagged with EGFP: XPA-EGFP (70 kD, D = 28 ± 5 µm²/s) and XPB-EGFP (>400 kD in TFIIH, D = 6.2 ± 4 pm²/s) (*21*), the observed D's were in agreement with the known size of the respective NER protein (complexes) (see Fig. 1G) *(20,21).* These findings argue against a model in which NER proteins are fixed to nuclear structures in the absence of damage.

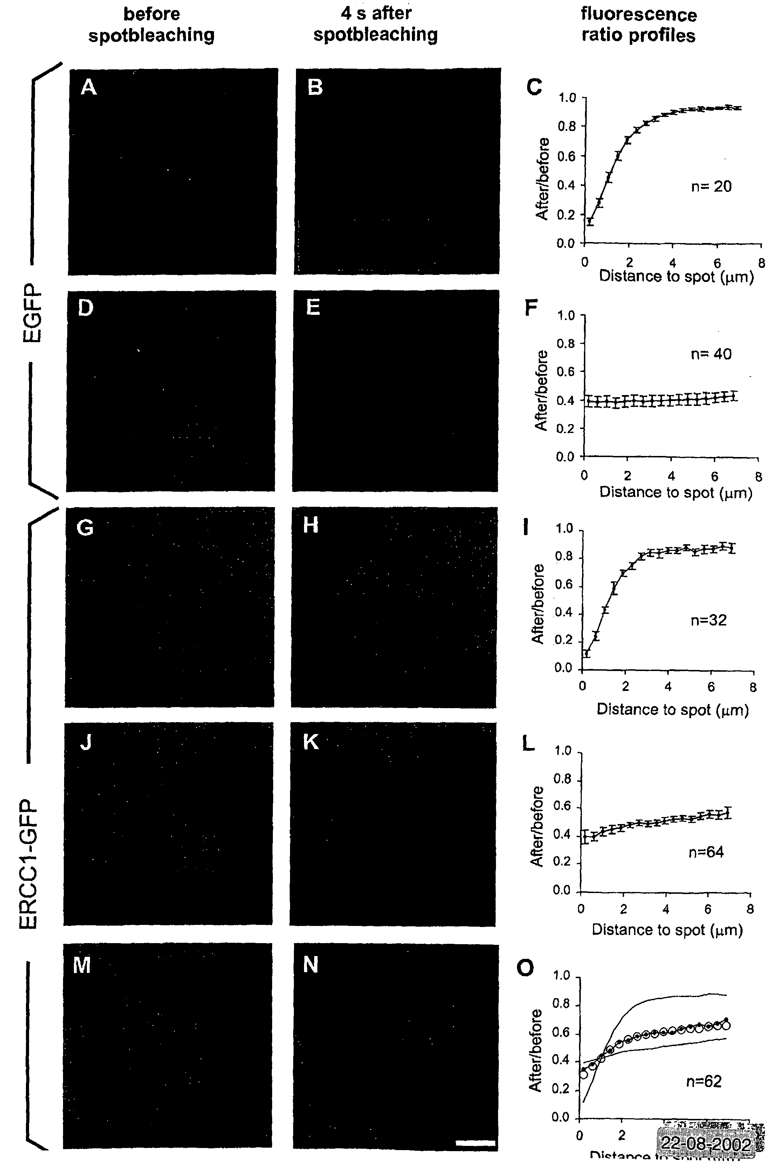

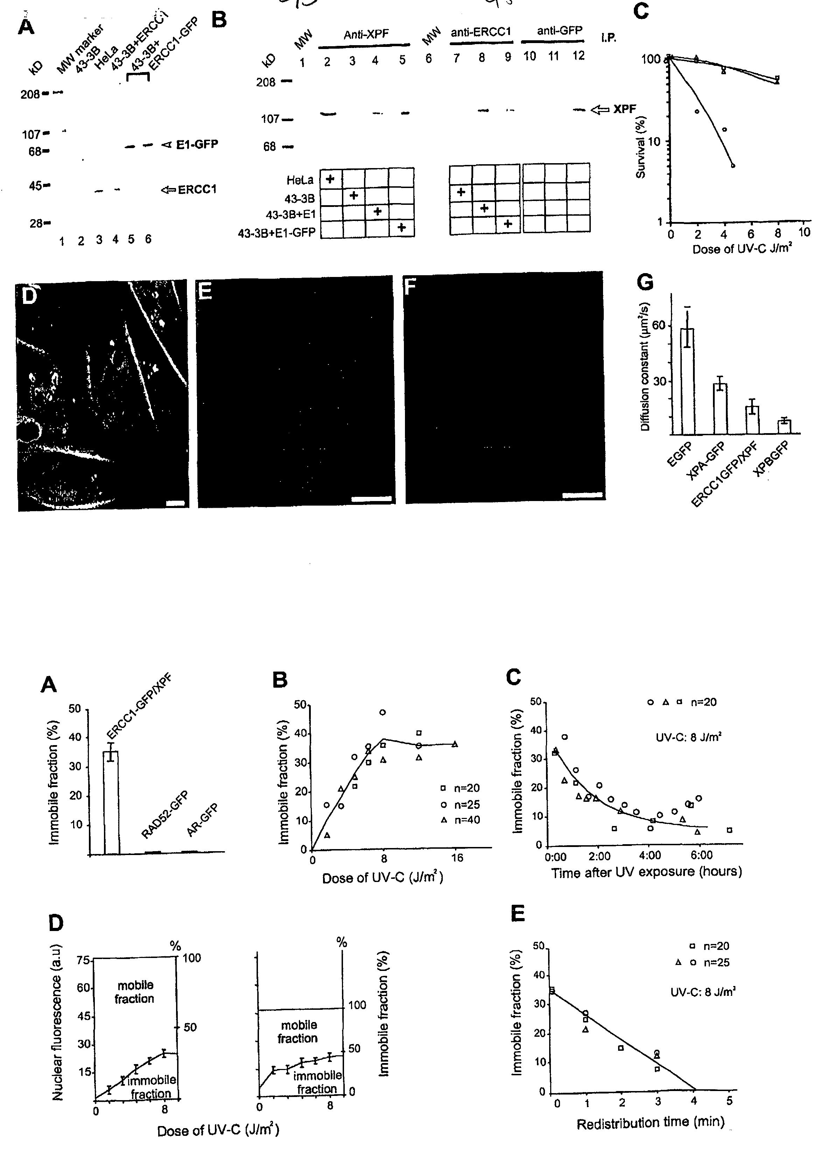

To study the effect of DNA damage on ERCC1-GFP/XPF mobility, we developed a technology based on FRAP *(19)* (designated FRAP-FIM) (see Fig. 2). Briefly, the fluorescence ratio of confocal sections before and 4 seconds after spot bleaching (which takes also 4 seconds) was plotted as a function of the distance to the bleached spot [fluorescence ratio profile (FRP)], (Fig. 2D). The method was validated with fixed and living SV-40 transformed human fibroblasts expressing EGFP. Fixed human fibroblasts expressing EGFP showed no bleaching outside the laser beam area (Fig. 3, A and B), and the experimentally obtained FRP (Fig. 3C) matched the theoretically predicted curve (Fig. 2D). In contrast, in living human fibroblasts, an average of 60% of the EGFP molecules in the nucleus passed through the laser cone volume during 4 s of spot bleaching and lost fluorescence. Four seconds after spot bleaching, the remaining fluorescence was uniformly redistributed within the nucleus, resulting in the predicted horizontal FRP (Fig. 3, D to F). Control experiments indicated that the FRAP-FIM procedure did not affect cell viability (15).

We then applied FRAP-FIM to the ERCC1-GFP expressing CHO-cells. The FRPs of fixed and living cells (Fig. 3, G to L) showed curves similar to those of fibroblasts expressing free EGFP (Fig'. 3, C and F). To investigate whether UV-induced NER altered the mobility of ERCC1-GFP/XPF, we applied FRAP-FIM to cells exposed to a UV-dose of 16 J/m² (Fig. 3, M to O). A significant fraction (35 ± 3 %) of ERCC1-GFP/XPF became immobilized within 5 minutes after UV irradiation, whereas tie remaining fraction showed a diffusion rate similar to that in untreated nuclei (*D*= 12 ± 5 µm²/s). This suggests that the mobile fraction, in the presence of damage is not incorporated into a holocomplex. UV-irradiation did not cause immobilization of EGFP, RAD52-EGFP (*22*), or GFP tagged androgen receptor (AR-GFP) (*23,15*), indicating that the ERCC1-GFP/XPF immobilization is not due to nonspecific UV-induced protein fixation (Fig. 4A).

To quantitate the immobilization, we applied FRAP-FIM to cells exposed to UV doses ranging from 1.6 to 16 J/m². The immobilized ERCC1-GFP/XPF fraction increased with UV dose, reaching a plateau of 38 ± 2 % at 8 J/m² (Fig. 4B), a dose known to saturate NER. Within 4 to 6 hours following UV exposure [the time required in CHO cells for removal of approximately 3.10⁵ 6-4PP photoproducts induced by 8 J/m² (*24*)] the immobilized fraction progressively decreased to background level (Fig. 4C). Since the amount of DNA damage decreases as a consequence of repair activity, these data strongly suggest that immobilization of ERCC1-GFP/XPF depends on the amount of damage present at any time. Consistent with this, the proportion of immobilized ERCC1-GFP/XPF was inversely related to the expression level of the protein (Fig. 4D). The parallels between repair kinetics and ERCC1-GFP immobilization, support the idea that immobilization reflects engagement of the complex in NER. The data in Fig. 4D also suggest that ERCC1-GFP/XPF is not rate-limiting for NER, but is present in excess. Thus, the observed ERCC1-GFP/XPF immobilization may reflect the kinetics of other NER factor (s).

To investigate how long ERCC1-GFP/XPF complexes are involved in repair, we quantitated the immobile fraction using FRAP-FIM with increasing redistribution times (Fig 2, E and F). In this assay, molecules that regain mobility as well as molecules that become immobilized in the period after spot bleaching (fluorescence redistribution time) do not contribute to the measured immobile fraction because they redistribute equally over the nucleus. Within ∼4 minutes after spot bleaching, >95% of the immobile fraction appeared to regain mobility (Fig. 4E). The linear decay suggests that ERCC1-GFP/XPF is immobilized for an average of ∼4 min. (estimated from the intersection with the time axis, Fig. 2, E and F). The inferred binding time of 4 minutes is consistent with the estimated time of a single NER event (*25*). These data suggest that ERCC1-GFP/XPF is assembled at sites of DNA damage with other NER constituents into immobile holocomplexes during one repair event. After release, ERCC1-GFP/XPF regains full mobility (D = 12 ± 5 µm²), indicating that it dissociates from the NER/DNA-complex.

Our results support a model in which ERCC1-GFP/XPF participates in NER in a distributive fashion. This proposed distributive action of ERCC1-GFP/XPF contrasts with the processive (DNA-scanning) mechanism that has been reported for NER proteins in prokaryotes (26). A distributive mechanism may be more efficient in eukaryotes because the genome is much larger and contains large stretches of noncoding DNA. However, we have not excluded the possibility that initial detection of DNA damage by the global genome NER initiator XPC/hHR23B (4) occurs in a processive fashion. In transcription-coupled repair, damage is probably detected by the elongating RNA polymerase (2), which by its nature is processive. Actual repair could take place at the nuclear matrix (*17,27*)

### Examples of other cells useful for testing in a method as provided by the invention for (im)mobility measurements in living cells

A number of stably transfected cell lines are listed that have been generated and characterised and that can be used for testing different classes of drugs. These transformants have been demonstrated to express physiological levels of functional endogenous, natural proteins tagged in vivo with GFP (or variants thereof). The panel of tester cell lines can be extended to include fluorescently or otherwise labelled gene products representative of all relevant pathways or processes within the cell or intact organisms. These include any signal transduction pathway, stress pathway, basic process such as transcription, replication (proliferation), translation, intracellular protein transport, differentiation, chromatin dynamics, excretion etc.

Also transformants expressing multiple differently labeled proteins can be obtained for analysis of effects on more than one process simultaneously. The cell lines described below can for example be employed for testing the effect of the following categories of agents or drugs: 1.

CHO lines expressing ERCC1-GFP. The ERCC1 protein forms a complex with the XPF product, and as such has a structure-specific endonuclease activity within the cell nucleus. The protein is simultaneously involved in two DNA repair pathways: Nucleotide Excision Repair (a versatile DNA repair mechanism dealing with a large variety of structurally unrelated lesions in the DNA) as well as Recombinational Repair of interstrand DNA cross links, which are very genotoxic. The protein is also implicated in mismatch repair and telomer maintenance. Inborn defects in the ERCC1/XPF complex are associated with two human syndromes: xeroderma pigmentosum (XP) and a rare form of a progeroid disorder characterized by hypersensitivity to UV and many other genotoxic agents, cancer predisposition, and premature ageing. The ERCC1-GFP-epressing cell line can be used for testing DNA lesions induced by UV light, numerous chemical compounds inducing bulky chemical adducts as well as interstand crosslinking agents and to find out whether the repair pathways in which ERCC1/XPF is implicated are activated. The last two categories of DNA damaging agents include a significant fraction of currently used chemotherapeutic agents. The cell line may also be utilised for testing agents causing lesions involving mismatch repair and for agents interfering with telomer maintenance, related to ageing.

Because the protein participates in multiple important cellular processes the cell line can also be a valuable tool for screening agents that interfere with any of the cellular genome stability processes in which the protein is engaged (such as antagonists). In addition a CHO cell line stably expressing GFP-tagged XPF is in preparation 2. Human SV40-transformed cell line expressing GFP-tagged XPB. The XPB DNA helicase is a subunit of the multi-functional TFIIH complex and it has a dual role in nucleotide excision repair and in the initiation of basal transcription by RNA polymerase II. Inherited pointmutations in this essential gene can give rise to three human disorders all displaying photosensitivity: the cancer-prone condition xeroderma pigmentosum (XP), the severe neurodevelopmental disease Cockayne syndrome (CS) in combination with XP, and the CS-like brittle-hair disorder trichothiodystrophy. The GFP-tagged XPB expressing cell line can be employed for the analysis of any agent inducing DNA damage that falls within the wide class of targets of the nucleotide excision repair machinery or any compound that interferes with this process. In addition it is suitable for the use of any agent that in some way affects the basal transcription initiation apparatus.

Similarly, a CHO cell line expressing the XPD helicase subunit of TFIIH tagged with GFP is in progress. 3. Human SV40-transformed cell line expressing GFP-tagged CSB or CSA protein. The CSA and CSB polypeptides are specifically engaged in transcription-coupled repair, that links any DNA repair system with an RNA polymerase blocked in front of a lesion. This process appears to be the main determinant responsible for the cytotoxic effect of a very wide category of genotoxic agents, that interfere with transcription. Recent evidence indicates that transcription-coupled repair may also be very crucial for contributing to the process of ageing. In addition, evidence suggests that the CSB product participates in transcription elongation for all RNA polymerase II transcribed (i.e. all structural) genes. Defects in the CSA and CSB proteins are responsible for the neurodevelopmental photosensitive disorder Cockayne syndrome (CS). The GFP-tagged CSA and CSB expressing cell lines can be utilized for analysis of any agent that disturbs transcription elongation, as well as agents or treatments that induce DNA damage, or interfere with transcription-coupled repair. The cell lines are also useful to find out whether such agents do interfere with transcription elongation or with the transcription-coupled repair processes. 4. Human SV40-transformed cell line expressing GFP-tagged XPA protein. The XPA protein plays a keyrole in the core of the nucleotide excision repair reaction, both in verifying the DNA damage as well as in organizing the repair apparatus around a recognized lesion. The protein gives a clean read-out for the pathway: it highly mobile when not engaged in repair events. Upon induction of DNA damage targeted by the nucleotide excision repair system it gets rapidly immobilized. Thus the cell line can be employed to find out whether a drug or agent induces damage that is a substrate for the nucleotide excision repair machinery and/or whether it interferes with this DNA repair process. 5. Human SV40-transformed cell line expressing GFP-tagged XPC. The unique property of the XPC protein is that it is able to trace a large variety of DNA lesions genome-wide. It is specifically implicated in the global genome subpathway of Nucleotide Excision Repair. To find out whether a drug or other agent induces DNA injury falling within the class recognized by the nucleotide excision repair system, or whether the agent interferes with the DNA damage recognition process, the GFP-tagged XPC cell line is the tool of choice. 6. CHO cell mutant expressing GFP-tagged XPG (in progress). 7. CHO cell line expressing GFP-tagged PCNA. The PCNA protein is crucial for a variety of nuclear processes notably, replication, various DNA repair mechanisms and recombination. Thus, this CHO line can be used to monitor the effect of a specific treatment or agents on a large set of cellular systems.

8. CHO cell line expressing GFP-tagged hRAD52. Homology-dependent recombination repair constitutes a major error-free mechanism to heal double strand breaks induced by X-rays and a number of chemical agents. For the repair of other types of DNA damage a double strand break represents an intermediate. Thus this process is very relevant in relation to radiotherapy and a number of cross linking chemotherapeutic agents (such as CisPlatin). Also double strand DNA breaks can be induced in several physiological processes such as V(D)J recombination in the immune system. To tag an important component acting within this pathway a CHO transformant was .generated stably expressing GFP-labeled hRAD52. This cell line acts as a reference line for analysis of agents inducing DNA injury relevant for this complex repair system.

Antagonists or any agent influencing this repair and recombination mechanism can be screened. 9. CHO cell line. expressing GFP-tagged androgen receptor. A well-characterized signal-transduction process is ligand induced transcriptional activation exerted by the androgen receptor, that moves from 'the cytoplasm to the nucleus upon administration of proper steroid hormones. This CHO tranformant expressing GFP-tagged Androgen receptor can be used as a read-out for activation of this transcription activation pathway.

The generation of many other cell transformants expressing GFP-tagged proteins involved in a variety of processes according to the above outlined procedures is a common skill in the art. One of the major advantages of this methodology as provided by the invention is that testing cells or cell lines, or tissue, organs or organisms comprising these allows to assess the effect of an agent at the level of its functionality.

### Figure legends

Fig. 1. Expression and functionality of ERCC1-GFP (*13*) in CHO 43-3B cells. Cell lines used: HeLa (repair proficient), 43-3B (ERCC1-deficient, UV-sensitive), 43-3B + ERCC1 (43-3B stably expressing nontagged ERCC1, repair-proficient), and 43-3B+ ERCC1-GFP (43-3B stably expressing ERCC1-GFP). (A) Immunoblot of whole cell extracts (WCE) (28) of transfected and control cell lines (10 µg protein per lane), probed with polyclonal anti-ERCC1. Arrow, untagged ERCC1; arrowhead, ERCC1-GFP. (B) Immunoblot, probed with anti-XPF (polyclonal), showing immunoprecipitations (IP) of WCE from the indicated cell lines. Immunocomplexes were bound to protein A beads and separated by SDS-PAGE. IPs were performed with anti-XPF (lanes 2 to 5), anti-ERCC1 (lanes 7 to 9) and anti-GFP (lanes 10 to 12). Lanes 1 and 6 contained markers for molecular size (as indicated). (C) Survival of 43-3B cells (circles), 43-3B + ERCC1-GFP cells (squares) and repair-proficient CHO-9 cells (triangles) after treatment with UV irradiation. (D) Combined phase-contrast (red) and fluorescence (green) image of ERCC1-GFP-transfected 43-3B cells. (E) Confocal image of ERCC1-GFP-transfected 43-3B cells, showing a homogeneous distribution of ERCC1. The lower nucleus shows a bright fluorescent spot (see text). (F) Confocal image of HeLa cells treated with polyclonal anti-ERCC1 antiserum (and stained with Cy-3 labeled secondary antibody), showing ERCC1 distribution and spots similar to the GFP fluorescence displayed in (E). (G) Diffusion constants of EGFP (27 kD) and three nucleotide excision repair proteins, XPA-GFP (70kD), ERCC1-GFP/XPF (163 kD) and XPB-GFP (>400 kD in TFIIH), showing a decrease of mobility with increasing molecular weight. Bars in D, E, and F: 5 µm.

Fig. 2. FRAP-FIM protocols for quantitation of immobilized molecules in living cells. (A to D) Procedure for measurement of the immobile fraction. (A) Theoretical prebleach fluorescence pattern of homogeneously distributed fluorophores in a confocal plane. (B) The laser beam (blue cones) is focused in the center of the nucleus during spot bleaching. A bleach time of 4 seconds (at 3% laser power) was used to allow mobile molecules to diffuse through the laser cone area. Small green spheres represent fluorescent molecules and the green disk represents the confocal plane. (C) Theoretical postbleach fluorescence patterns showing 100%, 0% and 33% immobile molecules. (D) Ratio of mean fluorescence intensity (MFI) of pre- and postbleach images (of the theoretical cases shown in C) plotted as a function of distance d to the laserspot. Fluorescence ratio is calculated as (MFI_{*d*}_{,post}-background)/(MFI_{*d*}_{,pre}-background). The mean immobilized protein fraction *p* (0 *< p <* 1) *of a sample* of cells is obtained by least square fitting of the mean fluorescence ratio profile FRPₛₐₘₚₗₑ(*d*) to the weighted sum FRP_{*ws*}(*d*) of the fluorescence ratio profiles of nuclei with containing immobile molecules only and nuclei containing mobile molecules only: FRP_{*ws*}(*d*) = *p* *FRP_{100%immobile}(*d*) + (1-*p*) * FRP_{0%immobile}(*d*). (E to F) Procedure for measurement of the average time that molecules stay immobile. (E) The fate of the molecules that are immobile at the time of bleaching (t=0) is monitored by recording postbleach images at increasing time intervals after bleaching (fluorescence redistribution time). Molecules that are released during this time interval will diffuse through the nucleus and no longer contribute to the measured immobile fraction. Molecules that become immobilized during the time interval also do not contribute to the measured immobile fraction. (F) Theoretical curves of the decay of the measured immobile fraction after bleaching determined by computer simulation of bleaching and diffusion in an ellipsoid volume. The solid line represents the theoretical decay of the measured immobile fraction when molecules are transiently immobile. The intersection with the time axis determines the average binding time, three minutes in this example. The dotted lines represent the decay of the measured immobile fraction when a fraction is not transiently immobile, but slowly diffuses through the nucleus.

Fig. 3. (A to F) Application of the FRAP-FIM procedure to human fibroblasts (XPCS2BA-SV40) expressing EGFP. (A and B) Pre- and postbleach confocal images of EGFP in a fixed fibroblast. Inset in (B) is a confocal z-scan (cross-section) through the nucleus at the site of spotbleaching (compare with the diagram in Fig. 2B). (C) Fluorescence ratio profile (FRP) of 20 fixed fibroblasts (compare with theoretical curve in Fig. 2D). (D and E) Pre- and postbleach confocal images of EGFP in a living fibroblast. (F) Fluorescence ratio profile of 40 living fibroblasts (G to 0) Effect of UV-irradiation on ERCC1-GFP/XPF mobility. The FRAP-FIM procedure was applied to ERCC1-GFP/XPF-expressing cells that were either fixed in 4% paraformaldehyde (G to I), untreated (J to L), or UV-irradiated (M to O). (G, J and M) Prebleach images. (H, K and N) Postbleach images of the same cell. Note that the UV-irradiated cell (N) displays a pattern that is intermediate between that of fixed (H) and untreated (K) cells (compare with Fig. 2C). (I, L and O) Fluorescence ratio profiles (FRPs, compare with Fig. 2D). (O) The FRP of UV irradiated (16 J/m²) cells (filled circles) optimally fits at *p* = 0.35±0.3 to the weighted sum (open circles, see legend of Fig. 2D) of fixed and untreated FRPs (solid lines), indicating that 35±3% of the ERCC1-GFP/XPF is immobile (the method of calculation corrects for the overall bleaching that is caused by taking the prebleach image. All images had the same magnification; the bar in O is 5 µm. Error bars in the graphs are two times standard error of the mean (SEM); n indicates the number of cells used for the FRPs

Fig. 4. Response of ERCC1-GFP/XPF to UV irradiation. (A) Immobile fraction of ERCC1-GFP/XPF, RAD52-GFP, and androgen receptor (AR-GFP) after UV irradiation (16 J/m²) (B) Immobile fraction of ERCC1-GFP/XPF plotted against UV dose. The curve represents the average of three independent experiments. Cells with low fluorescence levels were selected for these experiments to exclude possibly over-expressing cells from measurements. The number of cells n used for each experiment is indicated (C) Immobile fraction plotted against time after UV irradiation (8 J/m²). (D) Immobile ERCC1-GFP/XPF (white area) expressed as the percentage of prebleach mean fluorescence (grey+white area) plotted against UV dose. The cells that were used in the experiment represented in (B) were divided in two groups (of equal size) of cells with relatively high (left) and relatively low prebleach fluorescence (right). While the immobile fraction is significantly higher in low fluorescent cells, the actual amount of immobile ERCC1-GFP/XPF is the same in both groups. (E) Immobile fraction (8 J/m²) plotted against fluorescence redistribution time (compare with Fig. 2F). The intersection of the extrapolated curve with the time axis at four minutes, indicates that on average individual ERCC1-GFP/XPF molecules bind for four minutes.

### References and notes

1. E.C. Friedberg, G.C. Walker, W. Siede, *DNA Repair and Mutagenesis* (ASM Press, Washington D.C., 1995); R.D. Wood, *Annu. Rev: Biochem*. 65, 135 (1996); A. Sancar, *ibid*., p. 43; W.L. de Laat, N.G.J. Jaspers, J.H.J. Hoeijmakers, *Genes Dev*., in press.
2. P.C. Hanawalt, B.A. Donahue, K.S. Sweder, *Curr. Biol.* 4, 518 (1994).
3. D. Bootsma, K.H. Kraemer, J.E. Cleaver, J.H.J. Hoeijmakers, in *The Genetic Basis of Human Cancer,* B. Vogelstein and K. W. Kinzler, Eds. (McGraw-Hill, New York, 1998), pp245-274.
4. K. Sugasawa *et al., Mol. Cell* 2, 223 (1998).
5. J.H.J. Hoeijmakers, J-M. Egly, W. Vermeulen, *Curr. Opin. Genet. Dev.* 6, 26 (1996); K. Tanaka *et al., Nature* 348, 73 (1990); Coverley *et al*., *Nature 349,* 538 (1991).
6. W.L. de Laat *et al., Genes Dev.* 12, 2598 (1998).
7. A. O'Donovan, A.A.Davies, J.G. Moggs, S.C. West, R.D. Wood, *Nature* 371, 432 (1994)..
8. A. M. Sijbers. et *al., Cell* 86, 811 (1996); W.L. de Laat, E. Appeldoorn, N.G.J. Jaspers, J.H.J. Hoeijmakers, *J. Biol. Chem.* 273, 7835 (1998).
9. A. Aboussekhra *et al., Cell* 80, 859 (1995); D. Mu, et *al., J. Biol. Chem. 270, 2415 (1995).*
10. J.Q. Svejstrup *et al., Cell* 80, 21 (1995).
11. S. N. Guzder, P. Sung, L. Prakash, S. Prakash, *J*. *Biol. Chem.* 271, 8903 (1996); S. N. Guzder, P. Sung, L. Prakash, S. Prakash, *J. Biol. Chem.* 273, 6292 (1998).
12. D.C. Prasher, V.K. Eckenrode, W.W. Ward, F.G. Prendergast, M.J. Cormier, *Gene* 15, 111 (1992); M. Chalfie, Y. Tu, G. Euskirchen, W.W. Ward, D.C. Prasher, *Science* 263, 802 (1994).
13. Humanized GFP-S65T cDNA (Clonetech, Palo Alto) was cloned in frame and downstream of the ERCC1 open reading frame. The resultant cDNA was transfected, using lipofectin (Life Technologies), into ERCC1-deficient (UV-hypersensitive) CHO cells (43-3B). Stable transfectants were selected by growing cells in 800 µg/ml Geneticin (Life Technologies) and by exposing them to UV (4 J/m²) three times with one day interval. SV40-transformed human fibroblasts were transfected with pEGFP-N1 (Clonetech), selected with 300 µg/ml Geneticin and enriched by fluorescence activated cell sorting. All cell strains were cultured in RPMI+Hepes (Life Technologies) supplemented with 10% fetal calf serum, and maintained in a humidified 5% CO₂, 37 °C incubator.
14. A.J. van Vuuren *et al., EMBO J.* 12, 3693 (1993); M. Biggerstaff, D.E. Szymkowski, R.D Wood, *ibid.*, p.3685; C.-H. Park, T. Bessho, T. Matsunaga, A. Sancar, *J. Biol. Chem.* 270, 22657 (1995); A.M. Sijbers *et al., Nucleic Acids Res.* 24, 3370 (1996); T. Yagi, R.D. Wood, H. Takebe, *Mutagenesis* 12, 41 (1997).
15. Data not shown.
16. S. J. McCready and P.R. Cook, J. *Cell Sci.* 70, 189 (1984); J. Harless and R.R. Hewitt, *Mut., Res*. 183, 177 (1987); L.H.F. Mullenders, A.C. van Kesteren van Leeuwen, A.A. van Zeeland, A.T. Natarajan, *Nucleic Acids Res.* 16, 10607 (1988); M.S. Park, *Proc. Natl. Acad. Sci. USA* 93, 8368 (1996); A. Nakagawa *et al., J. Invest. Dermatol.* 110, 143 (1998).
17. D.A. Jackson, A.S. Balajee, L. Mullenders, P.R. Cook, J. *Cell Sci.* 107, 1745 (1994).
18. D.Axelrod, D.E. Koppel, J. Schlessinger, E. Elson, W.W. Webb, *Biophys. J.* 16, 1055 (1976); D.E. Wolf, *Methods* *Cell Biol.* 30, 271 (1989); N.B. Cole *et al., Science* 273, 797 (1996).
19. We measured *operational* diffusion constants as in [J. Ellenberg *et al., J. Cell Biol.* 138, 1193 (1997); C.L. Wey, M.A. Edidin, R.A. Cone, *Biophys. J.* 33, 225 (1981)]. In this method the data were fitted to a one-dimensional diffusion model. The operational diffusion coefficient of EGFP was in the same range as previously reported values, measured with conventional spot-FRAP (*18*) [R. Swaminathan, C.P. Hoang, A.S. Verkman, *Biophys. J.* 72, 1900 (1997)].
20. For a comparison between MW and D see [O. Seksek, J. Biwersi, A.S. Verkman, *J. Cell Biol.* 138, 131 (199')].
21. XPA and XPB were tagged with EGFP, respectively, at the NH2- and COOH-termini (unpublished data) and stably expressed in SV40-transformed XP-A and XP-B cells.
22. RAD52 is involved in homologous recombination repair of X-ray induced DNA-damage [T. Rijkers *et al., Mol. Cell Biol.* 11, 6423 (1998).]
23. The androgen receptor is involved in steroid-hormone dependent transcription regulation.
24. Global genome repair in rodent cells removes predominantly 6-4 photoproducts [D.L. Mitchel, C.A. Haipek, J.M Clarkson, *Mutat. Res.* 143, 109 (1985); D.L. Mitchell and R.S. Nairn, *Photochem. Photobiol.* 49, 805 (1989)].
25. Given that a nucleus contains ∼30,000 ERCC1-GFP/XPF-complexes (J.H.J. Hoeijmakers unpublished results), of which one sixth (averaged over the whole repair period , Fig. 4B) is involved in NER, we estimate that damage is repaired at a rate of 1,250 lesions per minute (1/6 times 30,000/4 minutes). Thus repair of 300,000 6-4PP lesions (16) induced by UV light (8 J/m²) would require ∼4 hours.
26. E.Y. Oh and L. Grossman, *J. Biol. Chem.* 264, 1336 (1989).
27. D.R. Koehler and P.C. Hanawalt, *Nucleic Acids Res.* 24, 2877 (1996).

Immunoblot analysis of total cell extracts (10µg) and immunoprecipitation of cell lysates were performed as described (8). A standard colony-survival procedure for assessment of UV-sensitivity (8) was applied to CHO-9 (parental repair-competent), 43-3B (ERCC1/repair-deficient), and 43-3B (ERCC1-GFP transfected) cells. Five days after UV-irradiation (Philips TUV-lamp), the cell survival rate (number of surviving treated colonies/ number of surviving untreated colonies) was plotted against UV dose.

## Claims

1. A method for determining the mobility of a certain analyte in a set volume comprising labelling said analyte with a laser-inducible moiety and subjecting a spot in a pre-determined plane through said set volume to a focused laser beam of relatively low intensity using low laser power wherein in the case of excitation at 488nm with a 60 mW Ar-laser a neutral filter passing <50% of the laserlight is used and when another lasertype and/or other filter is used these settings are adjusted accordingly to low laser power, for a relatively long time wherein a period of time is used that is required to bleach 30-70% of mobile labeled molecules, thereby inducing a measurable change in said moiety when exposed to said laser beam and determining the distribution of laser-exposed moiety in said plane.

2. A method according to claim 1 wherein said moiety is a fluorescent moiety.

3. A method according to claim 2 wherein said moiety, after excitation, bleaches upon exposure to said laser beam.

4. A method according to anyone of claims 1 to 3 wherein said distribution is determined relative to the distance of said laser-exposed moiety to said spot.

5. A method according to anyone of claims 1 to 4 wherein said distribution is determined in one or more concentric rings in said plane around said spot.

6. A method according to anyone of claims 1 to 5 wherein said distribution is determined a relatively long time after laser-exposure of said moiety.

7. A method according to anyone of claims 1 to 6 wherein said spot is subjected to a stationary confocal laser beam.

8. A method according to anyone of claims 1 to 7 wherein said set volume is exposed to said laser beam prior to subjecting said spot in said plane through said set volume to a focused laser beam.

9. A method according to anyone of claims 1 to 8 wherein said set volume is exposed to said laser beam after subjecting said spot in said plane through said set volume to a focused laser beam.

10. A method according to anyone of claims 1 to 9 wherein said analyte comprises an organic molecule.

11. A method according to claim 10 wherein said molecule comprises a protein.

12. A method according to anyone of claims 1 to 11 wherein said set volume is contained in a container.

13. A method according to claim 12 wherein said container is a cell or cell organelle.

14. A method according to claim 12 or 13 further comprising determining the mobility of said certain analyte in at least one other container.

15. A method according to claim 14 wherein in at least one container said analyte is rendered at least partly immobile.

16. A method according to 15 wherein said analyte is rendered immobile by fixation.

17. A method according to anyone of claims 1 to 16 wherein the distribution of laser-exposed moiety is determined relative to the pre-exposure distribution of said moiety.

18. A method according to anyone of claims 1 to 17 wherein the distribution of laser-exposed moiety is determined relative to post-exposure time.

19. A method according to anyone of claims 1 to 18 further comprising determining the mobility of at least one other analyte comprising labelling said other analyte with a second moiety.

20. A method according to anyone of claims 1 to 19 further comprising determining the mobility of said analyte in real time.

21. A method for testing biological activity of a candidate drug comprising subjecting a cell to said candidate drug and testing analyte mobility in said cell with a method according to anyone of claims 1 to 20.

22. A method for testing activity of a candidate catalyst comprising subjecting a set volume of a liquid system to said candidate catalyst and testing analyte mobility with a method according to anyone of claims 1 to 20.

23. A method according to claim 22 wherein said catalyst is an enzyme.

## Patentansprüche

1. Verfahren zum Ermitteln der Mobilität eines bestimmten Analyts in einem vorgegebenen Volumen, umfassend:
Kennzeichnen des Analyts mit einem Laser-induzierbaren Anteil und
Aussetzen einer Stelle in einer vorher festgelegten Ebene durch das vorgegebene Volumen einem fokussierten Laserstrahl von relativ geringer Intensität unter Einsatz geringer Laserenergie,
wobei im Fall einer Erregung bei 488 nm mit einem 60 mW Ar-Laser ein <50% des Laserlichts durchlassendes neutrales Filter verwendet wird und, wenn ein anderer Lasertypus und/oder ein anderes Filter verwendet wird, diese Vorgaben entsprechend eingestellt werden, um die Laserenergie zu verringern,
und zwar für eine relativ lange Zeit, wobei eine Zeitperiode verwendet wird, die benötigt wird, um 30 bis 70 % der mobilen gekennzeichneten Moleküle zu bleichen, wodurch eine messbare Veränderung in diesem Anteil induziert wird, wenn dieser dem Laserstrahl ausgesetzt wird, und die Verteilung des Laser-ausgesetzten Anteils in der Ebene ermittelt wird.

2. Verfahren gemäß Anspruch 1, bei welchem dieser Anteil ein fluoreszierender Anteil ist.

3. Verfahren gemäß Anspruch 2, bei welchem dieser Anteil durch die Laserstrahlaussetzung bleicht.

4. Verfahren gemäß einem der Ansprüche 1 bis 3,
bei welchem die Verteilung relativ zu dem Abstand von dem Laser-ausgesetzten Anteil zu der Stelle ermittelt wird.

5. Verfahren gemäß einem der Ansprüche 1 bis 4,
bei welchem die Verteilung in einem oder mehreren konzentrischen Ringen in der Ebene um die Stelle herum ermittelt wird.

6. Verfahren gemäß einem der Ansprüche 1 bis 5,
bei welchem die Verteilung eine relativ lange Zeit nach der Laseraussetzung dieses Anteils ermittelt wird.

7. Verfahren gemäß einem der Ansprüche 1 bis 6,
bei welchem die Stelle einem unveränderten, konfokalen Laserstrahl ausgesetzt wird.

8. Verfahren gemäß einem der Ansprüche 1 bis 7,
bei welchem das vorgegebene Volumen dem Laserstrahl ausgesetzt wird, bevor die Stelle in der Ebene durch das vorgegebene Volumen einem fokussierten Laserstrahl ausgesetzt wird.

9. Verfahren gemäß einem der Ansprüche 1 bis 8,
bei welchem das vorgegebene Volumen dem Laserstrahl ausgesetzt wird, nachdem die Stelle in der Ebene durch das vorgegebene Volumen einem fokussierten Laserstrahl ausgesetzt ist.

10. Verfahren gemäß einem der Ansprüche 1 bis 9,
bei welchem der Analyt ein organisches Molekül umfasst.

11. Verfahren gemäß Anspruch 10, bei welchem das Molekül ein Protein umfasst.

12. Verfahren gemäß einem der Ansprüche 1 bis 11,
bei welchem das vorgegebene Volumen in einem Behälter enthalten ist.

13. Verfahren gemäß Anspruch 12, bei welchem der Behälter eine Zelle oder ein Zellorganell ist.

14. Verfahren gemäß Anspruch 12 oder 13, ferner ein Ermitteln der Mobilität des bestimmten Analyts in wenigstens einem anderen Behälter umfassend.

15. Verfahren gemäß Anspruch 14, bei welchem in wenigstens einem Behälter der Analyt zumindest teilweise unbeweglich gemacht wird.

16. Verfahren gemäß Anspruch 15, bei welchem der Analyt durch Fixierung unbeweglich wird.

17. Verfahren gemäß einem der Ansprüche 1 bis 16,
bei welchem die Verteilung des Laser-ausgesetzten Anteils relativ zu der Verteilung des Anteils vor dem Aussetzen bestimmt wird.

18. Verfahren gemäß einem der Ansprüche 1 bis 17,
bei welchem die Verteilung des Laser-ausgesetzten Anteils relativ zu der Zeit nach dem Aussetzen bestimmt wird.

19. Verfahren gemäß einem der Ansprüche 1 bis 18,
ferner ein Ermitteln der Mobilität wenigstens eines anderen Analyts umfassend, umfassend ein Kennzeichnen des anderen Analyts mit einem zweiten Anteil.

20. Verfahren gemäß einem der Ansprüche 1 bis 19,
ferner ein Ermitteln der Mobilität des Analyts in Echtzeit umfassend.

21. Verfahren zum Testen biologischer Aktivität eines Arzneikandidaten, umfassend ein Aussetzen einer Zelle dem Arzneikandidaten und ein Testen der Analytmobilität in der Zelle mit einem Verfahren gemäß einem der Ansprüche 1 bis 20.

22. Verfahren zum Testen der Aktivität eines Katalysatorkandidaten, umfassend ein Aussetzen eines vorgegebenen Volumens eines liquiden Systems dem Katalysatorkandidaten und ein Testen der Analytmobilität mit einem Verfahren gemäß einem der Ansprüche 1 bis 20.

23. Verfahren gemäß Anspruch 22, bei welchem der Katalysator ein Enzym ist.

## Revendications

1. Procédé pour déterminer la mobilité d'un certain analyte dans un volume établi, consistant à marquer ledit analyte avec une fraction inductible par laser et à soumettre un point dans un plan prédéterminé via ledit volume établi à un faisceau laser focalisé d'intensité relativement faible en utilisant une puissance laser faible où, dans le cas d'excitation à 488 nm avec un laser Ar de 60 mW, un filtre neutre passant moins de 50% de la lumière laser est utilisé et quand un autre type de laser et/ou un autre filtre est utilisé, ces réglages sont ajustés en conséquence à une puissance laser faible, pendant un temps relativement long où une période de temps est utilisée qui est nécessaire pour blanchir 30-70% de molécules marquées mobiles, en induisant ainsi un changement mesurable dans ladite fraction quand elle est exposée audit faisceau laser et en déterminant la répartition de la fraction exposée au laser dans ledit plan.

2. Procédé selon la revendication 1, dans lequel ladite fraction est une fraction fluorescente.

3. Procédé selon la revendication 2, dans lequel ladite fraction, après excitation, blanchit lors de l'exposition audit faisceau laser.

4. Procédé selon l'une quelconque des revendications 1 à 3, dans lequel ladite répartition est déterminée par rapport à la distance de ladite fraction exposée au laser audit point.

5. Procédé selon l'une quelconque des revendications 1 à 4, dans lequel ladite répartition est déterminée dans un ou plusieurs anneaux concentriques dans ledit plan autour dudit point.

6. Procédé selon l'une quelconque des revendications 1 à 5, dans lequel ladite répartition est déterminée pendant un temps relativement long après l'exposition au laser de ladite fraction.

7. Procédé selon l'une quelconque des revendications 1 à 6, dans lequel ledit point est soumis à un faisceau laser cofocal immobile.

8. Procédé selon l'une quelconque des revendications 1 à 7, dans lequel ledit volume établi est exposé audit faisceau laser avant de soumettre ledit point dans ledit plan via ledit volume établi à un faisceau laser focalisé.

9. Procédé selon l'une quelconque des revendications 1 à 8, dans lequel ledit volume établi est exposé audit faisceau laser après avoir soumis ledit point dans ledit plan via ledit volume établi à un faisceau laser focalisé.

10. Procédé selon l'une quelconque des revendications 1 à 9, dans lequel ledit analyte comprend une molécule organique.

11. Procédé selon la revendication 10, dans lequel ladite molécule comprend une protéine.

12. Procédé selon l'une quelconque des revendications 1 à 11, dans lequel ledit volume établi est contenu dans un récipient.

13. Procédé selon la revendication 12, dans lequel ledit récipient est une cellule ou un organite cellulaire.

14. Procédé selon la revendication 12 ou 13 consistant de plus à déterminer la mobilité dudit analyte dans au moins un autre récipient.

15. Procédé selon la revendication 14, dans lequel, dans au moins un récipient, ledit analyte est rendu au moins partiellement immobile.

16. Procédé selon la revendication 15, dans lequel ledit analyte est rendu immobile par fixation.

17. Procédé selon l'une quelconque des revendications 1 à 16, dans lequel la répartition de la fraction exposée au laser est déterminée par rapport à la répartition de pré-exposition de ladite fraction.

18. Procédé selon l'une quelconque des revendications 1 à 17, dans lequel la répartition de ladite fraction exposée au laser est déterminée par rapport à un temps postexposition.

19. Procédé selon l'une quelconque des revendications 1 à 18, consistant de plus à déterminer la mobilité d'au moins un autre analyte consistant à marquer ledit autre analyte avec une seconde fraction.

20. Procédé selon l'une quelconque des revendications 1 à 19, consistant de plus à déterminer la mobilité dudit analyte en temps réel.

21. Procédé pour tester l'activité biologique d'un médicament candidat consistant à soumettre une cellule audit médicament candidat et à tester la mobilité de l'analyte dans ladite cellule avec un procédé selon l'une quelconque des revendications 1 à 20.

22. Procédé pour tester l'activité d'un catalyseur candidat consistant à soumettre un volume établi d'un système liquide audit catalyseur candidat et à tester la mobilité de l'analyte avec un procédé selon l'une quelconque des revendications 1 à 20.

23. Procédé selon la revendication 22, dans lequel ledit catalyseur est une enzyme.
